Europäisches Patentamt

European Patent Office

Office européen des brevets

(11) Numéro de publication: **0 377 547**

**A1**

(12) # DEMANDE DE BREVET EUROPEEN

(21) Numéro de dépôt: **90480007.5**

(22) Date de dépôt: **05.01.90**

(51) Int. Cl.5: **A61N 5/06, A61N 1/05, A61N 1/36, H04R 25/00**

(30) Priorité: **06.01.89 FR 8900227**

(43) Date de publication de la demande:
**11.07.90 Bulletin 90/28**

(84) Etats contractants désignés:
**AT BE CH DE DK ES FR GB GR IT LI LU NL SE**

(71) Demandeur: **SPECTEC S.A.**
**14 avenue Saint Augustin Impasse L. Gastaud**
**F-06200 Nice(FR)**

(72) Inventeur: **Houri, Hervé**
**29 avenue Colombo**
**F-06000 Nice(FR)**
Inventeur: **Ostrowsky, Daniel**
**1 Parc Liserb**
**F-06000 Nice(FR)**
Inventeur: **De Michelli, Marc**
**10 Chemin Mon Oustaou**
**F-06110 Cannet(FR)**

(74) Mandataire: **Hautier, Jean-Louis**
**Cabinet Hautier Office Méditerranéen de Brevets d'Invention et de Marques 24 rue Masséna**
**F-06000 Nice(FR)**

(54) **Prothèse optoélectronique de réhabilitation des handicapés neuromoteurs ou neurosensoriels utilisant au moins une source lumineuse et des fibres optiques pour la stimulation neurale ou tissulaire et son procédé de mise en oeuvre.**

(57) L'invention a pour objet une prothèse optoélectronique.

Le dispositif est composé d'un moyen faisant office de codeur et d'une ou plusieurs sources lumineuses qui convertissent les signaux amont du capteur en impulsions optiques, de moyens de transmission des impulsions optiques qui sont des fibres optiques (5), lesdites fibres optiques comportent à leur extrémité aval des moyens de couplage (6, 7, 8) pour la stimulation du nerf ou du tissu aval (9).

L'invention s'applique à la réhabilitation et au traitement des handicapés neuromoteurs ou neurosensoriels. .

Fig.1

## PROTHESE OPTOELECTRONIQUE DE REHABILITATION DES HANDICAPES NEUROMOTEURS OU NEURO-SENSORIELS UTILISANT AU MOINS UNE SOURCE LUMINEUSE ET DES FIBRES OPTIQUES POUR LA STIMULATION NEURALE OU TISSULAIRE ET SON PROCEDE DE MISE EN OEUVRE

L'invention a pour objet une prothèse optoélectronique de réhabilitation des handicapés neuromoteurs ou neurosensoriels utilisant une ou plusieurs sources lumineuses comme moyen d'information ou d'énergie et comme moyen de transmission des fibres optiques pour la stimulation neurale ou tissulaire et son procédé de mise en oeuvre.

L'idée de réaliser des prothèses pour pallier à une déficience du système nerveux n'est certes pas récente. Elle peut se concrétiser aujourd'hui en raison des progrès de la biologie et des technologies de la communication. En effet, si l'étude détaillée de la propagation de l'influx nerveux est encore du domaine des laboratoires, sa nature électrique est assez bien connue. Les progrès de l'électronique et de la micro-informatique permettent de concevoir des dispositifs miniaturisés très performants et très peu gourmands en énergie. Il apparait donc assez logique d'essayer de remplacer certaines parties déficientes du système nerveux (réseau électrique biologique), par un système électronique (réseau électrique artificiel). Cette technologie présente cependant un certain nombre de difficultés qui ont amené à proposer un système optoélectronique utilisant des lasers à semiconducteur et des fibres optiques qui sont décrits dans la suite de la description.

Les difficultés rencontrées lors de la mise au point de ces prothèses sont dues au phénomène de couplage électromagnétique. Par exemple, pour une pathologie rencontrée assez fréquemment chez les accidentés de la route : un tronçon de nerf endommagé. Dans ce cas, la possibilité de réaliser un pontage électrique est très attractive, mais elle se heurte à deux difficultés.

Premièrement, les fils électriques que l'on doit implanter pour raccorder les deux extrêmités saines du nerf vont se comporter comme autant d'antennes captant les signaux électromagnétiques qui environnent le patient, ce qui peut conduire à des réactions tout à fait imprévisibles de l'organisme.

Deuxièmement, ces fils se comportant comme des antennes, ils sont également capables de rayonner. Comme dans un nerf, les fibres indépendantes sont en très grand nombre, réaliser un pontage nécessite un grand nombre de fils électriques. Ceux-ci, malgré leur très faible diamètre, doivent être compactés de manière très dense pour que le système garde une taille compatible avec une implantation dans l'organisme. Les distances entre fils sont alors suffisamment faibles pour que le champ électromagnétique émis par un fil soit capté par ses plus proches voisins, ce qui conduit à un mélange d'information préjudiciable au bon fonctionnement du pontage.

Cet état de la technique, qui s'est développé essentiellement pour les prothèses auditives, peut être défini par les brevets suivants :

- FR-A-2.383.657 :

"Equipement pour prothèse auditive utilisant n électrodes implantées dans la cochlée en n points différents choisis pour permettre l'identification par le cerveau de n fréquences différentes, un implant récepteur couplé aux électrodes par l'intermédiaire d'un sélecteur et un émetteur extérieur couplé, par l'intermédiaire d'une inductance unique, à une inductance unique d'implant récepteur. L'émetteur comporte n filtres accordés sur n fréquences identifiables pour décomposer le signal d'information sonore recueilli par un microphone, ainsi que des moyens d'analyse séquentielle et des circuits permettant de former des signaux de trame, chaque signal de trame comportant au moins n impulsions dont les durées sont caractéristiques de l'énergie à transmettre à chacune des n électrodes. La transmission se fait par un signal alternatif haute fréquence modulée par les signaux de trame."

- FR-A-2.573.313 :

"Il comprend un support en matériau biocompatible isolant, des cavités ouvertes vers l'extérieur et ménagées dans le support de place en place, de manière à être séparées l'une de l'autre par du matériau isolant, des électrodes disposées dans certaines des cavités au moins et éloignées de la surface extérieure du support, de manière à ne pas être en contact direct avec le tissu à stimuler, ces électrodes étant reliées à des moyens propres à leur transmettre les signaux de stimulation appropriés."

-FR-A-2.586.932 :

"Ce dispositif comprend une électrode de stimulation et une électrode de masse, un générateur produisant un signal de stimulation fonction d'une loi prédéterminée, et des moyens pour engendrer consécutivement à chaque alternance d'une polarité donnée du signal de stimulation une alternance de neutralisation de réactions électrochimiques ayant une polarité opposée. Le générateur émet des impulsions de stimulation à courant constant ayant une charge électrique prédéterminée fonction de ladite loi et lesdits moyens produisent des impulsions de neutralisation, ayant chacune sensiblement la même charge électrique que l'impulsion de stimulation précédente."

- FR-A-2.586.928 :

"Cet appareil de stimulation neurale pour prothèse auditive à canal unique comprend un microphone apte à capter un signal sonore, un processeur électronique qui convertit ce signal sonore en sigaux électriques représentatifs au moins de l'énergie totale du signal sonore, et des moyens de transmission desdits signaux électriques à une électrode de stimulation d'un nerf auditif. Le processeur électronique comprend des moyens de traitement aptes à extraire d'un signal sonore de parole d'un locuteur une composante représentative du voisement de la parole du locuteur et à générer des signaux électriques représentatifs à la fois de l'énergie globale et du voisement du signal de parole."

Les dispositifs optoélectroniques, selon l'invention, décrits ci-après, se composent d'un ou plusieurs ensembles : sources lumineuses comme moyens d'information et d'énergie et fibres optiques comme moyens de transmission. Lesdites fibres optiques sont pourvues d'un moyen de couplage avec le tissu ou le nerf aval. Si ce système paraît a priori plus complexe que le système tout électrique, il présente de nombreux avantages.

Tout d'abord, il supprime tous les fils électriques de longueur notable et devient ainsi parfaitement insensible au rayonnement électromagnétique externe. Deuxièmement, les fibres optiques de diamètre très fin peuvent être compactées sans crainte de voir l'information passer de l'une à l'autre. Enfin, dans les nerfs neuromoteurs, la puissance électrique associée à l'influx nerveux est du même ordre de grandeur que celle nécessaire au fonctionnement des lasers à semi-conducteurs que l'on produit aujourd'hui. Cela permet donc d'envisager un fonctionnement parfaitement autonome (sans source de puissance extérieure) de la prothèse.

Les avantages ci-dessus décrits sont également fort appréciés dans le cas des prothèses neurosensorielles, même si l'argument des puissances n'est plus valable dans ce cas. Dans ce domaine, il existe aujourd'hui différents modèles d'oreilles artificielles qui ont donné des résultats très variables (voir l'état de la technique ci-dessus).

L'invention permet d'envisager la réalisation d'une oreille artificielle optoélectronique.

Les prothèses de réhabilitation permettent de donner un nouvel espoir à de nombreux handicapés neuromoteurs ou neurosensoriels. Les diverses technologies impliquées ont atteint un niveau de maturité tel que des réalisations efficaces sont envisageables dans un avenir très proche. Parmi les diverses approches possibles, il a été choisi la voie optoélectronique qui présente l'avantage de l'immunité électromagnétique, et qui, bien que nécessitant des composants spéciaux, fait appel à des technologies modernes (laser à semi-conducteurs, fibres optiques), mais éprouvées.

Toute stimulation neurale ou de tissus biologique, par exemple, musculaires, cardiaque, nodohisien venant à être déficiente ou absente soit par lésions, soit par diminution de conduction, soit par insuffisance du système excitateur amont peut trouver dans la présente invention un appareillage prothétique de stimulation ou de conduction.

Cet appareillage apporte à tout tissu biologique l'information et l'énergie utile à son fonctionnement, soit en assurant la liaison amont aval d'un tissu lésé ou peu fonctionnel dans une région située entre ces deux points (commande prothétique interne), soit en apportant une information d'une autre provenance (commande prothétique externe).

Dans le cas où il s'agit de fibres neurosensorielles déficientes car comprimées ou sectionnées en partie ou en totalité, le système peut soit réaliser le transfert des stimulations d'une section amont à une section aval, soit donner lieu aux stimulations neurales nécessaires pour éviter la dégénérescence des fibres nerveuses et des tissus situés en aval de la lésion.

Le dispositif ou prothèse avec son procédé assure les interfaces neuromusculaires, les interfaces sensorielles (auditives, olfactives, visuelles) les interfaces dans une neurorégulation, les interfaces agissant aux niveaux des centres somesthésiques et de la douleur, ainsi que les stimulations glandulaires. Le procédé par son mécanisme est applicable dans la conduction nodohisienne et dans la stimulation tissulaire cardiaque.

La présente invention présente la possibilité d'apporter au système neural ou tissulaire l'information et l'énergie utiles à son fonctionnement et à son excitation.

A cet effet, l'invention utilise les moyens ci-après : procédé de stimulations neurales ou tissulaires biologiques du type utilisant un ou plusieurs capteurs adéquats, fonction des signaux reçus pour la stimulation amont (par ondes, des signaux sonores, électriques, lumineux, chimiques, etc...), un ou plusieurs moyens faisant office de codeur, tels qu'un processeur électronique, convertit ces signaux en signaux adaptés aux moyens de transmission qui transmettent lesdits signaux adaptés à des moyens de couplage de stimulation du nerf ou du tissu aval, caractérisé par le fait que le moyen faisant office de codeur est adapté à une ou plusieurs sources lumineuses qui convertissent les signaux amont du capteur en impulsions optiques, que les moyens de transmission des impulsions optiques sont des fibres optiques et que lesdites fibres optiques comportent à leur extrémité aval des moyens de couplage pour la stimulation du nerf ou du tissu aval. La source lumineuse est un laser ou une diode électroluminescente.

Les moyens de couplage sont les extrémités elles-mêmes des fibres optiques protégées par un matériau biocompatible transparent, de forme adaptée, faisant office d'optique et ce, de manière à obtenir un couplage via des effets photoniques.

Le moyen faisant office d'optique peut être l'extrémité de la fibre.

Le moyen faisant office d'optique peut être le matériau biocompatible transparent.

Le moyen faisant office d'optique peut être un élément optique adéquat adapté.

Ces différents moyens peuvent être pris en combinaison ou non.

Les moyens de couplage sont des optogénérateurs de courant constitués par une cellule photovoltaïque réalisée sur un semi-conducteur et deux électrodes de stimulation disposées à l'extrémité de chaque fibre optique, de manière à ce que le couplage entre la fibre et le tissu ou nerf aval se fasse via des effets électroniques.

Les moyens de couplage qui utilisent des électrodes de stimulation sont pourvus d'un porte-électrodes qui assure la répartition spatiale.

Dispositif dans lequel le porte-électrodes est adapté à une oreille optoélectronique. Dans ce cas, la répartition des électrodes présente une identité suivant une génératrice ; les électrodes, en matériau biocompatible, sont des disques conducteurs percés suivant une répartition de trous concentriques de diamètre correspondant à celui des fibres optiques , qui permettent de recevoir les terminaisons suivant un ordre donné de manière à optimiser le remplissage de la cavité de fibres optiques ; les disques conducteurs sont isolés électriquement par des rondelles complètes ou des secteurs en matière isolante flexible, biocompatible, qui débordent des disques conducteurs pour former des anneaux de garde, l'ensemble reste flexible, compressible et extensible.

Dispositif dans lequel le porte-électrodes a une forme tronconique.

Dispositif dans lequel le porte-électrodes a une forme hélicoïdale.

Dispositif dans lequel le diamètre des disques conducteurs faisant office d'électrodes est constant.

Dispositif dans lequel le diamètre des disques conducteurs faisant office d'électrodes est décroissant depuis la base jusqu'au sommet, les disques les plus profonds étant de diamètre de plus en plus petit.

Les dessins ci-joints sont donnés à titre d'exemples indicatifs et non limitatifs. Ils représentent un mode de réalisation préféré selon l'invention. Ils permettront de comprendre aisément l'invention.

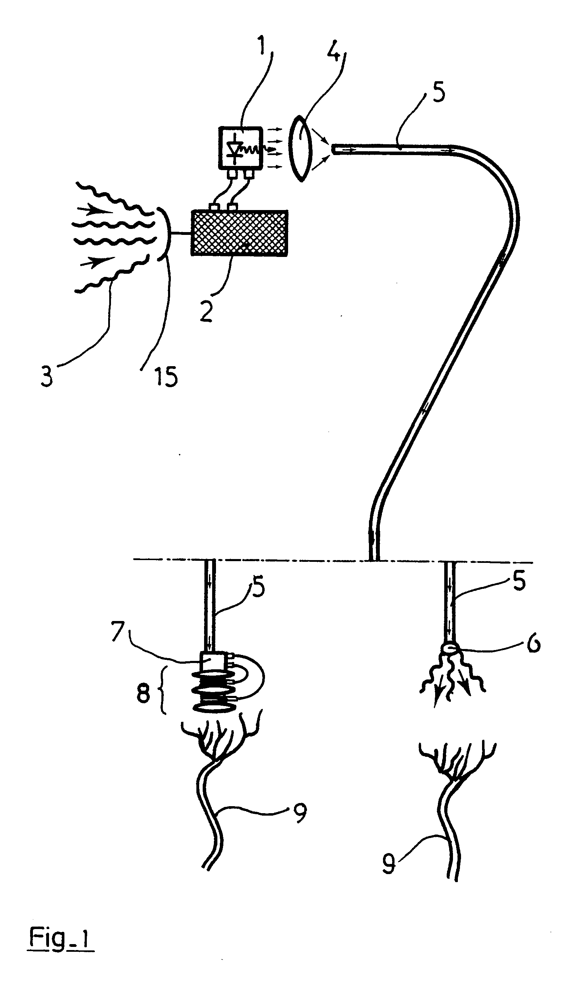

La figure 1 est une vue schématique du dispositif selon l'invention avec deux modes de réalisation au niveau du couplage des fibres optiques avec le système nerveux ou tissulaire aval.

La figure 2 est une vue schématique d'un dispositif appliqué à l'oreille de manière à réaliser une oreille optoélectronique.

La figure 3 est une vue schématique d'une terminaison annulaire.

La figure 4 est une vue en perspective d'un porte-électrodes mettant en évidence la répartition des fibres optiques, les disques conducteurs faisant office d'électrodes, les rondelles ou secteurs isolants, les espaces vides entre les électrodes.

La figure 5 est une vue du porte-électrodes représenté à la figure 4, vu de côté.

La figure 6 est une vue en perspective des rondelles ou moyens isolants flexibles et extensibles et de leur contre-moulage faisant office de moyens de protection.

La figure 7 est une vue en perspective des disques conducteurs faisant office d'électrodes, elle met en évidence la répartition des orifices sur les disques.

La figure 8 est une vue schématique de structuration des positions d'électrodes permettant une excitation du type damier.

La figure 9 est une vue en coupe du porte-électrodes selon la figure 8.

La figure 10 est une vue des électrodes constituées chacune par deux éléments séparés par un isolant ; dans cette vue les électrodes sont représentées de face.

La figure 11 est un schéma d'une prothèse neurologique.

A cet effet, l'invention utilise les moyens ci-après : procédé de stimulations neurales ou tissulaires biologiques du type utilisant un capteur adéquat 15, fonction des signaux reçus pour la stimulation amont 3 (par ondes, des signaux sonores, électriques, lumineux, chimiques, et...), un moyen faisant office de codeur 2, tel qu'un processeur électronique, convertit ou module ces signaux en signaux adaptés aux moyens de transmission 5 qui transmettent lesdits signaux adaptés à des électrodes de stimulation 8 du nerf ou du tissu aval 9. Le moyen faisant office de codeur 2 est adapté à une source lumineuse 1 telle qu'un laser semi-conducteur qui convertit les signaux amont du capteur 15 en impulsions optiques, que les moyens de transmission des impulsions optiques sont des fibres optiques 5. L'invention vise également le dispositif pour la mise en oeuvre du procédé.

Le dispositif est composé d'un moyen faisant office de codeur 2 qui module les signaux issus d'un capteur 15, qui capte les stimulations amont et qui est adapté à une source lumineuse telle qu'un laser semi-conducteur qui convertit les signaux amont du capteur 15 en impulsions optiques, de moyens de transmission des impulsions optiques qui sont des fibres optiques, lesdites fibres optiques comportent à leur extrémité aval des moyens de couplage 6, 7, 8 pour la stimulation du nerf ou du tissu aval 9. La source lumineuse 1 est un laser ou une diode électroluminescente.

Selon un mode de réalisation, les moyens de couplage 6 sont les extrémités elles-mêmes des fibres optiques protégées par un matériau biocompatible transparent, de forme adaptée, et d'un moyen faisant office d'optique 6 et ce, de manière à obtenir un couplage via des effets photoniques.

Selon un autre mode de réalisation, les moyens de couplage 7, 8 sont des optogénérateurs de courant 7 constitués par une cellule photovoltaïque réalisée sur un semi-conducteur et deux électrodes de stimulation 8 disposées à l'extrémité de chaque fibre optique 5, de manière à ce que le couplage entre la fibre 5 et le tissu ou nerf aval 9 se fasse via des effets électroniques.

Les moyens de couplage 7, 8 qui utilisent des électrodes de stimulation sont pourvus d'un porte-électrodes 16 qui assure la répartition spatiale.

Lesdites électrodes 8 sont disposées sur des porte-électrodes 16, dont la forme est adaptée au tissu ou au nerf particulier que l'on désire exciter.

La présente invention permet d'apporter au système neural l'information et l'énergie utiles à son excitation.

Cette énergie et cette information proviennent d'une ou plusieurs sources lumineuses 1 modulées 2 en fonction de la stimulation amont 3. Le rayonnement émis est ensuite couplé à l'aide d'une optique 4 dans une ou plusieurs fibres optiques 5 qui en assurent le transport. Le couplage des fibres optiques 5 avec le système nerveux aval 9 peut se faire de deux façons :
- soit l'extrémité de la fibre optique 5 est nue ou protégée par un matériau biocompatible transparent de forme adaptée faisant office d'optique 6 et le couplage se fait via des effets photoniques, par exemple photochimiques ou thermiques pris séparément ou à la fois.
- soit l'extrémité de la fibre optique 5 est équipée d'un optogénérateur de courant 7 constitué par une cellule photovoltaïque réalisée sur un semi-conducteur, silicium, arsénure de gallium, tellure de cadmium-mercure...ou tout autre matériau approprié et d'un système d'électrodes 8 réalisées en matériau conducteur biocompatible (platine, titane...) et le couplage se fait via des effets électroniques.

L'optogénérateur de courant 7 peut être du type mettant en oeuvre les moyens décrits dans le brevet FR-A-2.583.942 et ayant pour objet : "installation de télé-alimentation à guide optique".

Ces aspects de photoexcitation, de thermoexcitation, d'électrostimulation peuvent intervenir soit isolément, soit associés dans une répartition spatiale extraneurale, périneurale ou endoneurale, de même elle peut être extra, péri ou endotissulaire de manière plus générale. La stimulation amont, dont est fonction la modulation de la lumière, peut provenir, soit fibres nerveuses coupées ou partiellement lésées dont l'activité est détectée au travers d'un système de capteurs et d'interface électronique, soit d'une source externe dont l'information est totalement ou partiellement corrélée avec un système physique ou électrophysiologique d'excitation naturel ou de synthèse par micro-électronique ou micro-informatique. Ces systèmes physiques d'excitations peuvent être des ondes électromagnétiques, des ondes acoustiques, des ondes lumineuses, des ondes thermiques, des effluves stimulantes telles qu'olfactives ou des gradients physico-chimiques.

### Oreille optoélectronique

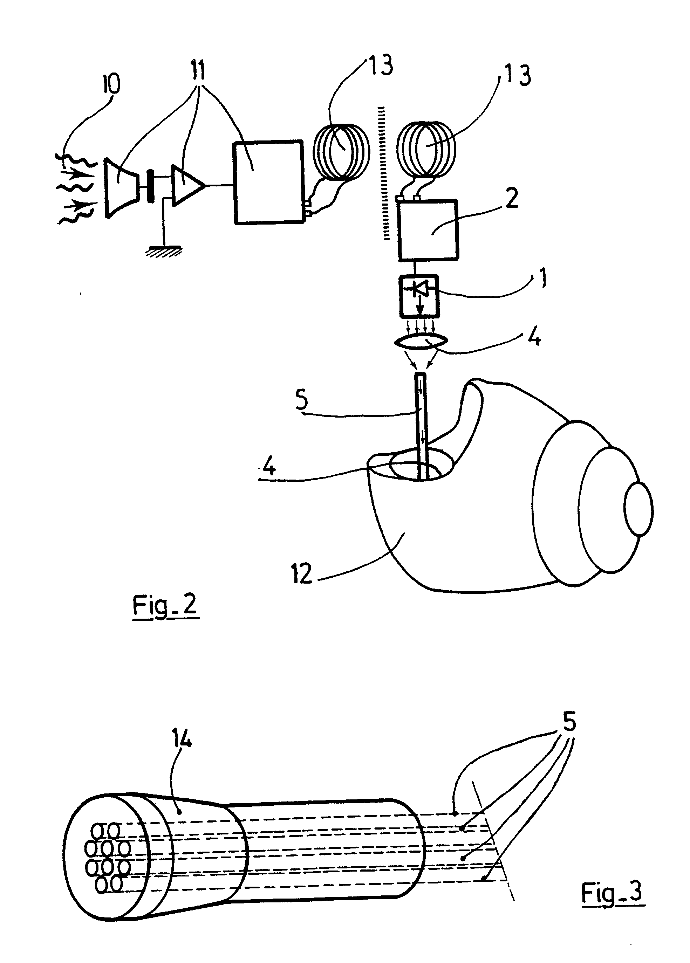

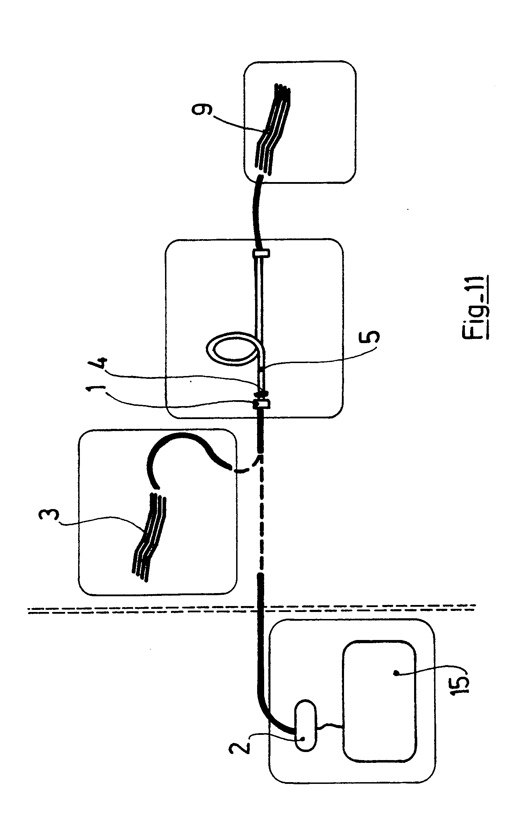

Le dispositif peut s'appliquer et être adapté de manière spécifique à l'oreille, de manière à permettre d'obtenir une oreille artificielle "oreille optoélectronique". La présente invention est applicable, bien entendu, dans ses principes partiels ou globaux à d'autres réhabilitations. Dans le cas de l'oreille optoélectronique (voir la figure 2), la stimulation externe est liée aux ondes acoustiques 10 dans le domaine des fréquences auditives et autres si cela est nécessaire. L'information contenue dans l'onde acoustique est analysée spectralement, numériquement ou analogiquement en ses composantes fréquencielles soit par une Transformée de Fourrier Rapide, soit par filtres (voir figure 11). Les paramètres résultants, intensité, fréquence, temps, barycentre des structures acoustiques (phonèmes et autres), aspects mélodies, voisement, etc... modulent des modulateurs ou codeurs 2 vers des sources lumineuses (diodes électroluminescentes, diodes lasers ou autres sources 1) dont les temps de réponse et les intensités émises sont compatibles avec la dynamique des paramètres analysés. L'énergie lumineuse correspondante est véhiculée par fibres optiques 5 jusqu'aux récepteurs neurosensoriels en l'occurrence, dans ce cas, l'organe de Corti 12, dont il est inutile de décrire les mécanismes neurosensoriels auditifs.

Dans cet exemple d'excitation des fibres nerveuses auditives, les fibres optiques peuvent produire, soit une excitation extracochléaire mono ou multicanaux, soit intracochléaire mono ou multicanaux. Dans ce dernier cas, on constatera les avantages de cette invention par rapport aux systèmes existants. Ces avantages sont explicités par la suite.

De cette invention, résulte un nouveau type d'implants cochléaire pour l'exemple rapporté, cet exemple n'est pas limitatif, les autres procédés d'application explicités au début conservent toutes leurs potentialités innovantes. En ce qui concerne l'implant cochléaire, il existe actuellement un grand nombre de systèmes développés à partir d'un réseau de conducteurs en fil de platine iridiée recouvert de téflon avec des électrodes au bout référencées principalement dans les brevets cités dans l'état de la technique.

Les fibres optiques 5 véhiculant l'information et l'énergie excitatrice sont totalement indépendantes entre elles. Il n'y a pas de couplage capacitifs interfils, (pas de couplage interfils par le milieu ionisé hydroconducteur où sont plongés ces fils, ou effets capacitifs de surfaces en regards). L'utilisation d'une excitation des fibres nerveuses de type photonique, ou l'utilisation d'un photodétecteur couplé à une paires d'électrodes excitatrices permet d'éviter les problèmes liés à l'accumulation des charges que l'on rencontre dans les systèmes implantés actuellement. De plus, notre système est de réalisation et de coût de revient très significativement inférieur aux systèmes existants.

Le procédé selon l'invention permet d'augmenter de manière importante le nombre d'électrodes implantées. La dimensions des fibres optiques, 100 microns de diamètre ou inférieur, leurs revêtements en Téflon (marque déposée), matière biocompatible, ou en Kevlar (marque déposée), en font un matériau implantologique idéal. L'augmentation de ce nombre d'électrodes implantées ou de fibres implantées permet une meilleure couverture et une meilleure disposition spatiale des implants en relation avec les terminaisons spécifiques le long de la rampe tympanique si celle-ci est utilisée comme voie d'accès. La présente invention, en utilisant les fibres optiques exploitées en parallèles, avec leur terminaisons optogénératrices de courants ou ses autres potentialités, permet, d'utiliser une plus grande et meilleure surface de contact entre le milieu hydroconducteur endolymphatique ou extracellulaire des fibres nerveuses.

Le dispositif pour la mise en oeuvre du procédé présente, comme on le verra en description, une configuration morphologique présentant des reliefs de garde périélectrodes ou périfibres en matière isolante comme par exemple le silicone médical biocompatible permettant d'éviter le contact direct de l'électrode ou de la zone active de stimulation avec les terminaisons nerveuses, qui, dans le cas d'électrodes métalliques ou de matériaux conducteurs, peut altérer ces terminaisons, les liquides ionisés assurent la conduction. Ces systèmes de garde peuvent être de formes toroïdales, semi-toroïdales, cylindriques, semi-cylindriques, annulaires, spiculées, ou autres formes. Dans chacun des cas, ces formes peuvent être présentes dans leur totalité ou en partie, voire supprimées.

Au contact métal des terminaisons nerveuses, des interactions peuvent apparaître avec altération de l'ensemble. Les systèmes de gardes en matériaux biocompatibles assurent une meilleure isolation des sections de liquides endolymphatiques en regard des terminaisons nerveuses.

Dans le cas où la terminaison de la fibre optique et du coupleur optogénérateur de courant ou d'énergie se fait sur des électrodes en platines iridiées ou autres matériaux conducteurs, titane par exemple, le système et le procédé d'innovation font que l'on peut avoir pour electrode une importante charge massique en platine iridiée avec des surfaces d'électrodes plus significativement importantes que dans les systèmes existants, ce qui augmente le potentiel d'activité de stimulation de l'électrode et la durée de vie de celle-ci qui est, comme nous le savons, appelée à une dissolution fonction du courant d'excitation utilisé et du milieu.

Couplage avec l'extérieur

Le couplage avec le milieu extérieur peut être classiquement fait à partir de couplage mutuel électromagnétique 13 ou parfois très simplement par aboutissement des fibres à l'extérieur (voir la figure 3).

Dans le cas de la figure 3, l'origine des fibres 5 est groupée en un faisceau, celui-ci est inséré dans une terminaison annulaire cylindrique 14 taraudée ou non, ou autre, en titane afin d'aboutir à l'extérieur soit par le conduit auditif externe, soit dans la région mastoïdienne au travers de ce tunnel 4 en titane dont l'ostéointégration ou l'insertion musculaire est banale. Le même procédé est applicable à d'autres tissus biologiques.

Cette approche permet l'accès à chacune de ces fibres 5 et une meilleure adaptation des différentes fibres 5 implantées à la pathologie considérée. L'optimisation de l'action de ces éléments se fait par des procédures externes autorisant des moyens plus importants que dans le cas de systèmes électroniques implantés sous-cutanés et intégrés dans la région osseuse mastoidïenne ou autre.

Dans un second temps après optimisation, il est possible de passer en structure totalement implantée, dont le descriptif, dans le cas de l'oreille optoélectronique, correspond à celui présenté dans la figure 2.


## Description du porte- électrodes 16

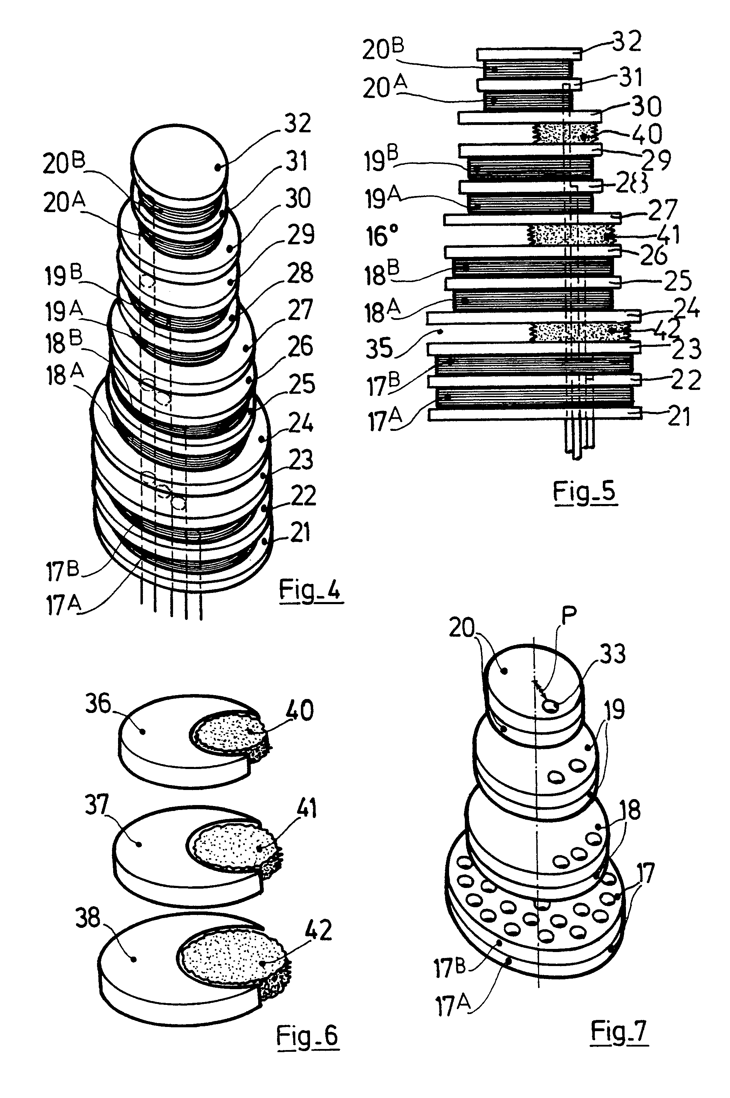

Une oreille optoélectronique, comme toutes les autres prothèses envisageables avec ce procédé, est donc composée de n fibres 5, chacune étant associée à ses éléments d'extrémité. En vue de transmettre correctement l'excitation au système nerveux ou musculaire aval, la répartition spatiale des extrémités des n fibres 5 et des électrodes éventuellement associées, est primordiale. Cette répartition est assurée par un porte-électrodes16 décrit ci-après dans les figures 4 à 8.

Dans ce porte-électrodes 16, la répartition des électrodes 17, 18, 19, 20 présente une identité suivant une génératrice dans le cas d'un modèle cylindrique ou tronconique ou hélicoïdal (figure 4). Dans le cas où l'on travaille en bipolaire, chaque électrode 17, 18, 19, 20 est constituée de deux éléments 17 A, 17 B ; 18 A, 18 B ; 19 A, 19 B ; 20 A, 20 B séparés par un isolant.

Une configuration spatiale donnée en figure 4 à 7 représente comment est assurée la liaison fibre optique 5 optogénérateur et électrodes 17, 18, 19, 20 en matériau conducteur biocompatible, platine iridiée ou titane (par exemple) (figures 4, 5, et 8 ).

Les électrodes 17, 18, 19, 20, en matériau ci-dessus cité, sont sous forme de disques conducteurs percés (figure 4 ), qui font donc office d'électrodes. Une répartition de trous concentriques 33 de diamètre correspondant à la terminaison de la fibre optique 5 permet de recevoir les terminaisons suivant un ordre donné, permettant d'optimiser le remplissage de la cavité (rampe tympanique par exemple) de fibres optiques 5. Dans la figure 7 est représenté un schéma figurant un type de remplissage. Le diamètre de ces disques peut être constant ou décroissant depuis la base jusqu'au sommet, les disques conducteurs ou électrodes 17 à 20 les plus profonds étant de diamètre de plus en plus petit, pour permettre une pénétration intracochléaire plus aisée.

La répartition des fibres 5 sur ces disques 17 à 20 (figure 7), se fait de manière que la première fibre 5, la plus éloignée, est associée à un trou 33 de position P, se trouvant le long de la circonférence du disque ou électrode 20 (figure 7). Il est possible d'avoir une ou deux fibres ou plusieurs fibres 5 sur un même disque, si n est ce nombre, le positionnement des fibres 5 sur les disques 17 à 20 est décalé de n trous 33 (figure 7) et ainsi de suite pour les disques électrodes 17 à 20.

Les dimensions respectives des différents diamètres des disques électrodes 17 à 20 vont réaliser un système conique donnant une meilleure flexibilité au porte électrode 16 décrit.

Les disques conducteurs font office d'électrodes 17 à 20, au nombre de deux par fibres 5 quand on travaille en bipolaire (ou éventuellement selon un autre mode de réalisation non représenté) deux portions du même disque 17 à 20, isolées électriquement, sont séparés par un moyen faisant office d'isolant 21 à 32, tel qu'une surface ou un disque très fin, en matière isolante bicompatible obtenue, par exemple, par moulage outout autre dépôt (figure 6). Ces isolants 21 à 32 débordent des disques électrode 17 à 20 pour constituer des anneaux ou systèmes de gardes (figure 4) et (figure 5) évitant le contact direct entre l'électrode et le système nerveux, comme cela a été décrit précédemment.

Chaque électrode 17 à 20 (figure 4 et 5) est donc prise en sandwich entre deux éléments isolants 21 à 32 (figures 4 et 5).

Deux électrodes 17 (17 A, 17B) et 18 (18 A, 18 B) seront séparées par un espace libre 35 (figures 4 et 5) ou partiellement rempli de matériau isolant 40, 41, 42, de consistance très molle, rendant le système flexible, compressible, extensible à la manière d'un accordéon (figures S et 6). Un exemple de structure de cet espace 35 est réalisé par moulage suivant les moules 36, 37, 38 donnés en figure 6. Il pourra être protégé de différentes manières telles que, par exemple, par un contre-moulage 36, 37, 38 représenté en figure 6. De manière croissante avec la position des disques électrodes 17 à 20, une injection croissante et un espace croissant d'injection de matière isolante biocompabile 40, 41, 42 depuis la fibre associée au disque électrode 20 le plus petit jusqu'au disque électrode le plus grand 17, assurent par ailleurs une zone d'étanchéité des jonctions de la fibre ou des différentes fibres. Les éléments de moulage 36, 37, 38, correspondant à ceux représentés à la figure 6, donnent lieu aux espaces 35 des figures 4 et 5.

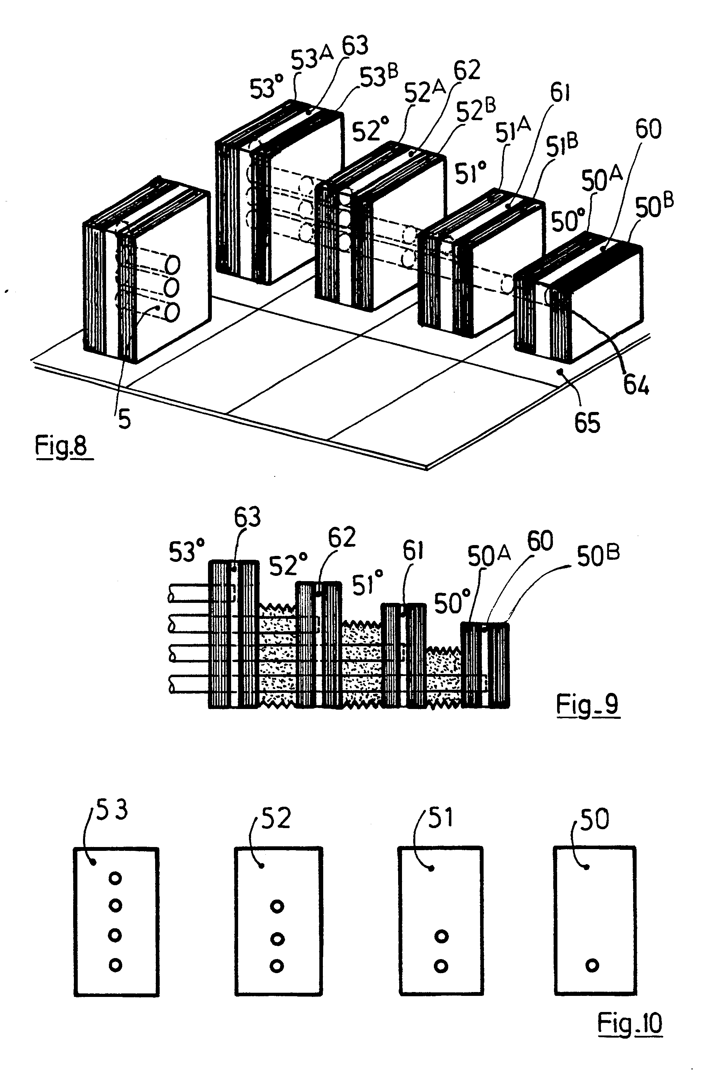

Cet exemple décrit pour l'excitation endocochléaire dont la forme est bien particulière, est directement appliqué à l'excitation neurale ou tissulaire quelconque de géométrie plane ou de surface gauche quelconque. Dans ce cas, les figures 8 et 9 montrent un exemple de structuration des positions d'électrodes 50, 51, 52, 53 permettant une excitation de type damier (figure 8), les fibres optiques 5 avec leurs terminaisons et les générateurs photovoltaïques sont représentées à la figure 8. Chacune de ces électrodes 50, 51, 52, 53 est constituée par deux éléments 50 (50A, 50B), etc...53 (53A, 53B)- (fig. 8,) séparés par un isolant 60, 61, 62, 63 (fig.8), celui-ci isole    pôles électriques de l'optogénérateur 64. Etan   donné que ledit optogénérateur est constitué lui-même de pôles positif et négatif isolés électriquement, d'autres aspects d'excitation sont à considérer : ils relèvent de l'application ou de l'implantation directe des fibres 5 dans le tissu biologique avec une répartition qui est fonction des stimulations nécessaires au tissu 65 (figure 8).

La figure 10 montre, comme pour l'exemple d'utilisation endocochléaire, une répartition spatiale des fibres.

La figure 11 est un schéma d'une prothèse neurologique.

Le dispositif optoélectronique est chargé de transporter l'information et de la transmettre au système nerveux aval. L'information provient de l'influx nerveux amont, dans le cas d'un pontage, ou d'une commande extérieure, dans le cas de l'oreille artificielle. Le procédé et son dispositif de mise en oeuvre peuvent être étendus à d'autres modèles de prothèse.

## REFERENCES

1. source lumineuse optique
2. source lumineuse modulée ou codeur
3. stimulation amont
4. une optique
5. fibres optiques
6. moyens de couplage optique via des effets photoniques
7. optogénérateur de courant
8. électrode de stimulation via des effets électroniques
9. système nerveux aval
10. ondes acoustiques
11. filtres
12. organe de CORTI
13. couplage électromagnétique
14. tunnel ou terminaison annulaire
15. capteur amont
16. porte-électrodes
17 à 20. disques conducteurs percés d'orifices faisant office d'électrode
21 à 32. moyens isolants
33. trous de position P
36, 37, 38. éléments de moulage faisant office de moyens de protection
40, 41, 42. matière isolante flexible et extensible (type accordéon)
50, 51, 52, 53. électrodes permettant une excitation du type damier
60, 61, 62, 63. isolant
64. optogénérateur
65. tissu

**Revendications**

1. Procédé de stimulations neurales ou tissulaires biologiques du type utilisant un ou plusieurs capteurs adéquats (15), fonctions des signaux reçus pour la stimulation amont (3), un ou plusieurs moyens faisant office de codeur, tels qu'un processeur électronique, convertissent ces signaux en signaux adaptés aux moyens de transmission qui transmettent lesdits signaux adaptés à des électrodes de stimulation du nerf ou du tissu aval (9), caractérisé par le fait

que l'on utilise un moyen faisant office de codeur (2), adapté à une ou plusieurs sources lumineuses (1) qui convertissent les signaux amont du capteur en impulsions optiques, que les moyens de transmission des impulsions optiques sont des fibres optiques (5), et que lesdites fibres optiques (5) comportent à leur extrémité des moyens de couplage pour la stimulation du nerf ou du tissu aval (9).

2. Procédé selon la revendication 1, caractérisé par le fait

que la source lumineuse (1) est un laser ou une diode électroluminescente

3. Procédé selon la revendication 1, caractérisé par le fait

que les moyens de couplage (6) sont les extrémités elles-mêmes des fibres optiques (5), protégées par un matériau biocompatible transparent de forme adaptée, et d'un moyen faisant office d'optique (6) et ce, de manière à obtenir un couplage via des effets photoniques.

4. Procédé selon la revendication 1, caractérisé par le fait

que les moyens de couplage (7, 8) sont des optogénérateurs de courant (7) constitués par une cellule photovoltaïque, réalisée sur un semi-conducteur et deux électrodes de stimulation (8), disposées à l'extrémité de chaque fibre optique (5), de manière à ce que le couplage entre ladite fibre (5) et le tissu ou nerf aval (9) se fasse via des effets électroniques.

5. Procédé selon la revendication 4, caractérisé par le fait

que les moyens de couplage (7, 8) utilisent des électrodes (8), lesdites électrodes sont montées sur un porte-électrodes (16) qui assure la répartition spatiale.

6. Dispositif pour la mise en oeuvre du procédé, selon la revendication 1, caractérisé par le fait

qu'il comporte un moyen faisant office de codeur (2), adapté à une ou plusieurs sources lumineuses (1) qui convertissent les signaux amont du capteur en impulsions optiques, que les moyens de transmission des impulsions optiques sont des fibres optiques (5), et que lesdites fibres optiques (5) comportent à leur extrémité des moyens de couplage pour la stimulation du nerf ou du tissu aval (9).

7. Dispositif selon la revendication 6, caractérisé par le fait que dans le cas d'un couplage où les fibres optiques (5) sont équipées à leur extrémité d'un moyen optogénérateur de courant et où ledit couplage s'effectue avec des électrodes de stimulation (8), via des effets électriques, la répartition spatiale des extrémités des n fibres optiques et des n électrodes est assurée par un porte-électrodes (16).

8. Dispositif selon la revendication 6, dans lequel le porte-électrodes est adapté à une oreille,caractérisé par le fait que dans ce cas, la répartition des électrodes présente une identité suivant une génératrice ; les électrodes, en matériau biocompatible, sont des disques électrodes conducteurs percés(17 à 20) suivant une répartition de trous concentriques (33) de diamètre correspondant à la terminaison de chaque fibre optique (5), qui permettent de recevoir les terminaisons suivant un ordre donné de manière à optimiser le remplissage de la cavité de fibres optiques (5) ; les disques électrodes conducteurs (17 à 20) sont isolés électriquement par des rondelles complètes ou des secteurs en matière isolante flexible (21 à 32), biocompatible, qui débordent des disques électrodes conducteurs (17 à 20) pour former des anneaux de garde, l'ensemble reste flexible, compressible et extensible.

9. Dispositif selon la revendication 8, caractérisé par le fait que le porte-électrodes a une forme tronconique.

10. Dispositif selon la revendication 8, caractérisé par le fait que le porte-électrodes a une forme hélicoïdale.

11. Dispositif selon la revendication 8, caractérisé par le fait que le diamètre des disques conducteurs (17 à 20) faisant office d'électrodes est constant.

12. Dispositif selon la revendication 8, caractérisé par le fait que le diamètre des disques conducteurs, faisant office d'électrodes est décroissant depuis la base jusqu'au sommet, les disques les plus profonds étant de diamètre de plus en plus petit.

13. Dispositif selon la revendication 6, caractérisé par le fait que le faisceau de fibres optiques est recouvert de matériaux biocompatibles pour transférer l'information et l'énergie nécessaire au système neural ou aux tissus biologiques.

14. Dispositif selon la revendication 6, caractérisé par le fait qu'il comporte des reliefs en matériaux isolants biocompatibles périélectrodes ou périfibres pour adapter le contact de ces fibres et de ces électrodes aux nécessités physiologiques du tissu biologique.

15. Dispositif selon la revendication 6, caractérisé par le fait que les disques conducteurs font office d'électrodes (17 à 20) et sont séparés par un moyen faisant office d'isolant (21 à 32), tel qu'une surface ou un disque très fin, en matière isolante biocompatible ; ces isolants (21 à 32) débordent des disques électrodes (17 à 20) pour constituer des anneaux ou systèmes de garde, évitant le contact direct entre l'électrode et le système nerveux.

16. Dispositif selon l'une quelconque des revendications 6 ou 8, caractérisé par le fait que deux électrodes (17, 17 A, 17 B) et (18, 18 A, 18 B) sont séparées par un espace libre (35) ou partiellement rempli de matériau isolant (40, 41, 42) de consistance très molle, rendant le système flexible, compressible, extensible à la manière d'un accordéon.

17. Dispositif selon la revendication 16, caractérisé par le fait que les moyens isolants flexibles et extensibles (40, 41, 42), type accordéon, peuvent être protégés par des contre-moulages de protection (36, 37, 38) qui viennent occuper l'espace laissé libre.

Fig_1

Fig_2

Fig_3

Fig_4

Fig_5

Fig_6

Fig_7

Fig.8

Fig_9

Fig.10

Fig_11

Office européen
des brevets

**RAPPORT PARTIEL
DE RECHERCHE EUROPEENNE**
qui selon la règle 45 de la Convention sur le brevet
européen est considéré, aux fins de la procédure ultérieure,
comme le rapport de recherche européenne

Numéro de la demande

EP 90 48 0007

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| P,X | GB-A-2 206 797 (BARR & STROUD LTD.) <br><br> * En entier * | 1,2,4-7,13 | A 61 N 5/06 <br> A 61 N 1/05 <br> A 61 N 1/36 <br> H 04 R 25/00 |
| A | US-A-3 900 034 (KATZ) <br><br> * Colonne 2, ligne 12 - colonne 3, ligne 19; colonne 4, lignes 24-38 * | 1,2,3,6 | |
| A | BE-A- 897 652 (UDELL) <br><br> * En entier * | 1,2,6 | |
| A | EP-A-0 215 726 (UNIVERSITY OF MELBOURNE) <br><br> * Page 2, ligne 21 - page 4, ligne 3; page 4, ligne 28 - page 7, ligne 2 * | 8,16 | **DOMAINES TECHNIQUES RECHERCHES (Int. Cl.4)** <br><br> A 61 N <br> A 61 B <br> A 61 F <br> H 04 R |

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes: 6-17

Revendications ayant fait l'objet de recherches incomplètes: 1-5

Revendications n'ayant pas fait l'objet de recherches:

Raison pour la limitation de la recherche:

Méthode de traitement chirurgical ou thérapeutique du corps humain ou animal (voir art. 52(4) de la convention sur le brevet européen)

| Lieu de la recherche | Date d'achèvement de la recherche | Examinateur |
|---|---|---|
| LA HAYE | 20-04-1990 | LEMERCIER |